(19) Europäisches Patentamt

European Patent Office

Office européen des brevets

(11) **EP 1 092 156 B1**

(12) **EUROPÄISCHE PATENTSCHRIFT**

(45) Veröffentlichungstag und Bekanntmachung des Hinweises auf die Patenterteilung:
**15.11.2006 Patentblatt 2006/46**

(21) Anmeldenummer: **00941875.7**

(22) Anmeldetag: **15.04.2000**

(51) Int Cl.:
***G06F 19/00*** *(2006.01)*

(86) Internationale Anmeldenummer:
**PCT/DE2000/001217**

(87) Internationale Veröffentlichungsnummer:
**WO 2000/067031 (09.11.2000 Gazette 2000/45)**

(54) **VERFAHREN ZUR ERMITTLUNG OSTEOPOROTISCHER PROZESSE**

METHOD OF DETERMINING OSTEOPOROTIC PROCESSES

PROCEDE POUR DEPISTER DES PROCESSUS OSTEOPOROTIQUES

(84) Benannte Vertragsstaaten:
**AT BE CH CY DE DK ES FI FR GB GR IE IT LI LU MC NL PT SE**

(30) Priorität: **30.04.1999 DE 19919982**

(43) Veröffentlichungstag der Anmeldung:
**18.04.2001 Patentblatt 2001/16**

(73) Patentinhaber: **PES GESELLSCHAFT FÜR MEDIZINISCHE DIAGNOSESYSTEME MBH**
**04416 Leipzig-Markkleeberg (DE)**

(72) Erfinder:
• **BITTERLICH, Norman**
  **D-09113 Chemnitz (DE)**
• **LÖSER, Thomas**
  **D-04105 Leipzig (DE)**

(74) Vertreter: **Seerig, Dieter**
**Patentanwälte**
**Findeisen Hübner Neumann Seerig**
**Pornitzstrasse 1**
**09112 Chemnitz (DE)**

(56) Entgegenhaltungen:
EP-A- 0 557 663      WO-A-95/08115
WO-A-96/12187

• GARNERO ET AL.: "Biochemical markers of bone turnover" ENDOCRINOLOGY AND METABOLISM CLINICS OF NORTH AMERICA, Bd. 27, Nr. 2, Juni 1998 (1998-06), Seiten 303-323, XP000938127 US in der Anmeldung erwähnt
• RIJS ET AL.: "Biochemical markers of bone trunover to monitor bone response to post menopausal hormone replacement therapy" OSTEOPOROSIS INTERNATIONAL, Bd. 5, Nr. 4, 1995, Seiten 276-280, XP000955635

**Beschreibung**

Die Erfindung betrifft ein Verfahren zur Ermittlung osteoporotischer Prozese.

Mit zunehmendem Alter nimmt im Wechselspiel von Knochenabbau- und Kaochenaufbauprozessen der Verlust an Knochensubstanz zu. Im Allgemeinen kann dieser Verlust durch Lebensweise und Ernährung derart ausgeglichen werden, daß es zu keiner Einschränkung der Lebensqualität kommt. Ein überdurchschnittlicher Verlust (Osteoporose) führt jedoch zu gesundheitlichen Beeinträchtigungen und erhöhtem Knochenbruchrisiko, so daß eine Behandlung notwendig wird. Zur Beschreibung der Knochendichte müssen Knochendichtemessungen (Osteodensitometrie) durchgeführt werden. Mit Methoden der Photonenabsorption bzw. der Computertomographie erfolgt die Messung der Knochenmineraldichte an der Speiche (Radius), am Oberschenkelknochen (Femur) oder an der Lendenwirbelsäule (Vertebra lumbalis). Allgemein anerkannte, alters- und geschlechtsspezifische Grenzwerte erlauben eine Zuordnung der konkreten Meßwerte zur osteoporotischen oder zur nichtosteoporotischen Situation. Die osteodensitometrischen Methoden erfüllen die Anforderungen an ein Screening-Verfahren nicht. Sie haben sich bislang - trotz aller Einschränkungen aufgrund fehlender Alternativen - lediglich in der Überwachung etabliert. Die Methoden sind aufwendig, mit außergewöhnlichen Belastungen für den Patienten verbunden und als nichtstandardisierte Verfahren in den Ergebnissen nicht vergleichbar. Zudem kann eine einzige Messung ohne einen individuellen Bezugswert den Status nicht ausreichend erfassen. Um im Screening-Verfahren die aufwendigen osteodensitometrischen Messungen zu umgehen, werden Indikatoren aus dem Zellanteil, aus der organischen Matrix oder aus den anorganischen Knochenbestandteilen zur Erfassung von osteoporotischen Prozessen (Bonemarker) gesucht, die einfacher zu ermitteln sind. Die Hoffnungen haben sich bislang nicht erfüllt. Die Bonemarker sind als Einzelwerte zu unspezifisch.

Die WO 96 12187 A (HORUS THERAPEUTICS), 25. April 1996 (1996-04-25) offenbart eine Methode zur Ermittlung osteoporotischer Prozesse, bei der mindestens drei Bonemarker gemessen werden, dessen Messwerte infolge durch einen Klassifikationsalgorithmus, basierend auf linearer Regression der unnormierten Daten, verarbeitet werden und das Ergebnis als bewertende Aussage zu osteoporotischen Prozessen ausgegeben wird.

Aufgabe der Erfindung ist es, ein Verfahren zur Ermittlung osteoporotischer Prozesse zu finden, bei dem die körperliche Belastung des Patienten und der technische Meß- und Analyseaufwand verringert sind.

Erfindungsgemäß wird die Aufgabe dadurch gelöst, dass aus Serum oder aus Urin im Labor Parameter ermittelt werden, die mit dem Knochendichteverlust assoziieren und als Bonemarker bekannt sind zur Ermittlung von osteoporotischen Prozessen verwendet werden, wobei

a) Laborwerte von mindestens drei Bonemarkern, wie Osteocalcin (Oc), Parathormon (PTH) und Alkalische Phosphatase (AP), gemessen und als Datenblock abgelegt werden,

b) die Messwerte auf Wertbeschränkungen nach unten ($R_u$) bzw. nach oben ($R_o$), für:

| | | |
|---|---|---|
| PTH | $R_u$ 10 ng/l | $R_o$ 65 ng/l |
| AP (männlich) | $R_u$ 70 U/l | $R_o$ 175 U/l |
| AP (weiblich) | $R_u$ 55 U/l | $R_o$ 170 U/l |
| Oc (männlich | $R_u$ 0..ng/l | $R_o$ 13 ng/l |
| Oc (weiblich) | $R_u$ 0..ng/l | $R_o$ 11 ng/l, |

normiert werden :

$$M^*_k = \left( \frac{M_k - R_{u,k}}{R_{o,k} - R_{u,k}} \right) \quad (k=1,...,K)$$

c) Die normierten Messwerte mit einem Klassifikationsalgorithmus (KA), bestehend aus

- der Fuzzifizierung über die normierten Bereiche mittels Dreieckfunktionen zu "niedrig", "erhöht" und "hoch"
- deren Verknüpfung mittels symmetrischen Regelwerkes und
- der Defuzzifizierung mittels Schwerpunktmethode über "keine", "gering" "deutlich" und "sicher",

verarbeitet werden und

d) das Ergebnis der Verarbeitung als eine bewertende Aussage zu osteoporotischen Prozessen in

- deutliches Anzeichen,
- sicheres Anzeichen sowie

- zwischen geringen und deutlichen Anzeichen

ausgegeben wird.

Im folgenden Ausführungsbeispiel wird die Erfindung näher erläutert. Als Bonemarker werden Osteocalcin (Oc), Parathormon (PTH) und Alkalische Posphatase (AP) genutzt. Für diese Bonemarker sind methodenabhängig folgende Referenzbereiche bekannt (vgl.: Froreich, A. u.a.: Analysenverzeichnis - Mikrobiologische Diagnostik - Klinische Indikatoren. Hamburg 1996).

| Bonemarker | Ru | Ro |
|---|---|---|
| PTH | 10 ng/l | 65 ng/l |
| AP (männlich) | 70 U/l | 175 U/l |
| AP (weiblich) | 55 U/l | 170 U/l |
| Oc (männlich) | - | 13 ng/l |
| Oc (weiblich) | - | 11 ng/l |

Es folgen die Arbeitsschritte:

a) die ermittelten Meßwerte von 3 Patienten werden jeweils in einem Datenblock zusammengefaßt:

| Pat.-Nr. | Geschlecht | Alter | Ocin ng/l | PTH in ng/l | AP in U/l |
|---|---|---|---|---|---|
| 990137 | weiblich | 57 | 10,45 | 34,5 | 105 |
| 990156 | männlich | 63 | 12,98 | 45,6 | 180 |
| 990201 | weiblich | 68 | 9,35 | 23,4 | 195 |

b) Die Meßwerte werden bezüglich der bekannten Referenzbereiche unter Beachtung des Geschlechtes normiert:

$$\left( M^*_k \ = \ \frac{M_k - R_{u,k}}{R_{o,k} - R_{u,k}} \right).$$

| Pat.-Nr. | Geschlecht | Alter | Ocin ng/l | PTH in ng/l | AP in U/l |
|---|---|---|---|---|---|
| 990137 | weiblich | 57 | 0,95 | 0,45 | 0,48 |
| 990156 | männlich | 63 | 1,00 | 0,65 | 1,05 |
| 990201 | weiblich | 68 | 0,85 | 0,24 | 1,22 |

Als Klassifikationsalgorithmus wird ein Fuzzy-System mit folgender Konfiguration genutzt:

| Eingangsgrößen | | | |
|---|---|---|---|
| Variable | Terme | | |
| normierter Oc-Wert | niedrig | erhöht | hoch |
| normierter PTH-Wert | niedrig | erhöht | hoch |
| normierter AP-Wert | niedrig | erhöht | hoch |

Die Fuzzifizierung der Eingangsgrößen erfolgt einheitlich mittels Dreiecksfunktion im Bereich -0,5 bis 1,5.

Die Freiheitsgrade des Systems liegen in der Festlegung dieser Dreiecksfunktion. Durch Änderungen aufgrund der Ergebnisanpassung an Patientendaten mit bekannten Status können die Zahlenwerte der Ausgangsvariablen verändert werden, ohne die prinzipielle Reihenfolge wesentlich zu beeinflussen.

Die Ausgangsgröße ist eine Singleton-Variable mit den Termen "keine" (-1), "geringe" (0), "deutliche" (1) und "sichere" (2) Anzeichen für osteoporotische Prozesse.

Das Regelwerk besteht aus 27 Regeln der folgenden Form. Die Verknüpfung erfolgt nach Max-Min-Methode.

| Eingang 1 | Eingang 2 | Eingang 3 | Ausgang |
|---|---|---|---|
| niedrig | niedrig | niedrig | keine |
| niedrig | niedrig | erhöht | keine |
| niedrig | niedrig | hoch | geringe |
| niedrig | erhöht | niedrig | keine |
| niedrig | erhöht | erhöht | geringe |
| niedrig | erhöht | hoch | geringe |
| niedrig | hoch | niedrig | geringe |
| niedrig | hoch | erhöht | geringe |
| niedrig | hoch | hoch | deutliche |
| erhöht | niedrig | niedrig | keine |
| erhöht | niedrig | erhöht | geringe |
| erhöht | niedrig | hoch | geringe |
| erhöht | erhöht | niedrig | geringe |
| erhöht | erhöht | erhöht | geringe |
| erhöht | erhöht | hoch | deutliche |
| erhöht | hoch | niedrig | geringe |
| erhöht | hoch | erhöht | deutliche |
| erhöht | hoch | hoch | deutliche |
| hoch | niedrig | niedrig | geringe |
| hoch | niedrig | erhöht | geringe |
| hoch | niedrig | hoch | deutliche |
| hoch | erhöht | niedrig | geringe |
| hoch | erhöht | erhöht | deutliche |
| hoch | erhöht | hoch | deutliche |
| hoch | hoch | niedrig | deutliche |
| hoch | hoch | erhöht | deutliche |
| hoch | hoch | hoch | sicher |

Für die Defuzzifizierung wird die übliche Schwerpunktmethode verwendet.

c) Setzt man die normierten Werte in das Fuzzy-System ein, erhält man folgende Ausgangswerte:

| Pat.-Nr. | Ausgangswert |
|---|---|
| 990137 | 0,85 |
| 990156 | 1,14 |
| 990201 | 0,60 |

d) Für die Aussage bezüglich des osteoporotischen Prozesses lassen sich folgende Bewertungen ableiten:

| Pat.-Nr. | Bewertung |
|---|---|
| 990137 | deutliche Anzeichen |
| 990156 | sichere Anzeichen |
| 990201 | zwischen geringen und deutlichen Anzeichen |

Für eine konkrete Entscheidung können die Patienten 990137 und 990201 verglichen werden, wobei das Ergebnis des ersten Patienten auffälliger für osteoporotische Prozesse als das Ergebnis des zweiten Patienten ist.

**Patentansprüche**

1. Verfahren zur Ermittlung osteoporotischer Prozesse, **dadurch gekennzeichnet, dass** aus Serum oder aus Urin im Labor Parameter ermittelt werden, die mit dem Knochendichteverlust assoziieren und als Bonemarker bekannt sind zur Ermittlung von osteoporotischen Prozessen verwendet werden, wobei

a) Laborwerte von mindestens drei Bonemarkern, wie Osteocalcin (Oc), Parathormon (PTH) und Alkalische Phosphatase (AP), gemessen und als Datenblock abgelegt werden,
b) die Messwerte auf Wertbeschränkungen nach unten ($R_u$) bzw. nach oben ($R_o$), für:

| | | |
|---|---|---|
| PTH | $R_u$ 10 ng/l | $R_o$ 65 ng/l |
| AP (männlich) | $R_u$ 70 U/l | $R_o$ 175 U/l |
| AP (weiblich) | $R_u$ 55 U/l | $R_o$ 170 U/l |
| Oc (männlich | $R_u$ 0..ng/l | $R_o$ 13 ng/l |
| Oc (weiblich) | $R_u$ 0..ng/l | $R_o$ 11 ng/l, |

normiert werden :

$$M^{\star}_k = \left( \frac{M_k - R_{u,k}}{R_{o,k} - R_{u,k}} \right) \quad (k=1,\ldots,K)$$

c) Die normierten Messwerte mit einem Klassifikationsalgorithmus (KA), bestehend aus

- der Fuzzifizierung über die normierten Bereiche mittels Dreieckfunktionen zu "niedrig", "erhöht" und "hoch"
- deren Verknüpfung mittels symmetrischen Regelwerkes und
- der Defuzzifizierung mittels Schwerpunktmethode über "keine", "gering", "deutlich" und "sicher",

verarbeitet werden und
d) das Ergebnis der Verarbeitung als eine bewertende Aussage zu osteoporotischen Prozessen in

- deutliches Anzeichen,
- sicheres Anzeichen sowie
- zwischen geringen und deutlichen Anzeichen

ausgegeben wird.

**Claims**

1.  Method of determining osteoporotic processes, **characterized in that** parameters which are associated with the bone density loss and are known as bone markers are determined from serum or from urine in the laboratory and are used to determine osteoporotic processes,

    a) laboratory values of at least three bone markers such as osteocalcin (Oc), parathormone (PTH) and alkaline phosphatase (AP) being measured and stored as a data block,
    b) the measured values being normalized as follows:

$$M^*_k = \left( \frac{M_k - R_{u,k}}{R_{o,k} - R_{u,k}} - \right) \quad (k=1,\ldots,K)$$

    to minimum value restrictions ($R_u$) and maximum value restrictions ($R_o$) for:

    | | | |
    |---|---|---|
    | PTH | $R_u$ 10 ng/l | $R_o$ 65 ng/l |
    | AP (male) | $R_u$ 70 U/l | $R_o$ 175 U/l |
    | AP (female) | $R_u$ 55 U/l | $R_o$ 170 U/l |
    | Oc (male) | $R_u$ 0..ng/l | $R_o$ 13 ng/l |
    | Oc (female) | $R_u$ 0..ng/l | $R_o$ 11 ng/l |

    c) the normalized measured values being processed with the aid of a classification algorithm (KA) comprising

    - the fuzzification over the normalized regions by means of triangular functions to "low", "increased" and "high"
    - the combination thereof by means of a symmetrical rule set, and
    - the defuzzification by means of centroid methods via "none", "weak", "clear" and "reliable", and

    d) the result of the processing being output as an evaluative statement in,

    - a clear indication,
    - a reliable indication, and
    - an indication between weak and clear.

**Revendications**

1.  Procédé de dépistage de processus d'ostéoporose, **caractérisé en ce que** sur du sérum ou de l'urine, on détermine en laboratoire des paramètres associés à la perte de densité osseuse et connus comme marqueurs osseux, pour les utiliser pour dépister des processus d'ostéoporose, et dans lequel :

    a) les valeurs de laboratoire d'au moins trois marqueurs osseux, par exemple l'ostéocalcine (OC), la parathormone (PTH) et la phosphatase alcaline (AP) sont mesurées et conservées en mémoire sous forme de blocs de données,
    b) les valeurs de mesure sont normalisées par rapport à des limites inférieures ($R_u$) et supérieures ($R_o$) de :

    | | | |
    |---|---|---|
    | PTH | $R_u$ 10 ng/l | $R_o$ 65 ng/l |
    | AP (homme) | $R_u$ 70 U/l | $R_o$ 175 U/l |
    | AP (femme) | $R_u$ 55 U/l | $R_o$ 170 U/l |
    | Oc (homme) | $R_u$ 0.. ng/l | $R_o$ 13 ng/l |
    | Oc (femme) | $R_u$ 0.. ng/l | $R_o$ 11 ng/l |

$$M^{*}_{k} = \left(\frac{M_k - R_{u,k}}{R_{o,k} - R_{u,k}}\right) \quad (k=1,\ldots,K)$$

c) les valeurs de mesure normalisées sont traitées à l'aide d'un algorithme de classification (KA) constitué de la façon suivante :

- fixation floue dans les plages normalisées, au moyen de fonctions triangulaires, en "bas", "accru" et "haut",
- association de ces valeurs au moyen d'un ensemble de règles symétriques et
- par une méthode à centre de gravité, "défloutage" en "rien", "peu", "net" et "certain" et

d) le résultat du traitement est délivré sous la forme d'une conclusion d'évaluation des processus d'ostéoporose, par :

- " indication nette",
- "indication sûre" ainsi que
- "indication comprise entre faible et nette".